# EUROPEAN PATENT APPLICATION

(11) **EP 1 609 802 A1**
(43) Date of publication of application: **28.12.2005**
(21) Application number: 04714461.3
(22) Date of filing: 25.02.2004
(51) Int. Cl.: C07K 14/47, C12N 15/12, A61K 38/00, A61P 35/00

(54) **TRANSCRIPTIONAL FACTOR INDUCING APOPTOSIS IN CANCER CELL**

(30) Priority: 26.02.2003 JP 2003048658
(71) Applicant: Japan Science and Technology Agency, Kawaguchi-shi, Saitama 332-0012 (JP); JAPAN as represented by PRESIDENT OF NATIONAL CANCER CENTER, Chuo-ku, Tokyo 104-0045 (JP)
(72) Inventor: TAYA, Yoichi, National Cancer Center, Tokyo 104-0045 (JP); ENARI, Masato, NationalCancer Center, Tokyo 104-0045 (JP)
(74) Representative: Wilson Gunn
(86) International application number: PCT/JP2004/002238
(87) International publication number: WO 2004/076483

(57) **Abstract**

A new method for treating cancer by inducing apoptosis exclusively in cancer cells and killing them is provided. The present invention relates to a transcriptional factor, comprising p53 or a mutated type p53, wherein one or more amino acids are deleted, substituted or added with respect to the amino sequence of p53, and clathrin heavy chains and having an activity to induce apoptosis of cancer cells. The transcriptional factor enhances the transcriptional activity of p53AIP1 promoter and induces apoptosis of cancer cells.

SEQUENCE LISTING

<110>  Japan Science and Technology Agency

<120>  A transcriptional factor inducing apoptosis of cancer cells

<130>  FS04-408PCT

<160>  11

<170>  PatentIn version 3.3

<210>  1
<211>  393
<212>  PRT
<213>  Homo sapiens

<400>  1

<210>  2
<211>  1675
<212>  PRT
<213>  Homo sapiens

<400>  2

Met Ala Gln Ile Leu Pro Ile Arg Phe Gln Glu His Leu Gln Leu Gln
1               5                   10                  15

Asn Leu Gly Ile Asn Pro Ala Asn Ile Gly Phe Ser Thr Leu Thr Met
            20                  25                  30

Glu Ser Asp Lys Phe Ile Cys Ile Arg Glu Lys Val Gly Glu Gln Ala
        35                  40                  45

Gln Val Val Ile Ile Asp Met Asn Asp Pro Ser Asn Pro Ile Arg Arg
    50                  55                  60

Pro Ile Ser Ala Asp Ser Ala Ile Met Asn Pro Ala Ser Lys Val Ile
65                  70                  75                  80

Ala Leu Lys Ala Gly Lys Thr Leu Gln Ile Phe Asn Ile Glu Met Lys
                85                  90                  95

Ser Lys Met Lys Ala His Thr Met Thr Asp Asp Val Thr Phe Trp Lys
            100                 105                 110

Trp Ile Ser Leu Asn Thr Val Ala Leu Val Thr Asp Asn Ala Val Tyr
        115                 120                 125

His Trp Ser Met Glu Gly Glu Ser Gln Pro Val Lys Met Phe Asp Arg
    130                 135                 140

His Ser Ser Leu Ala Gly Cys Gln Ile Ile Asn Tyr Arg Thr Asp Ala
145                 150                 155                 160

Lys Gln Lys Trp Leu Leu Leu Thr Gly Ile Ser Ala Gln Gln Asn Arg
                165                 170                 175

Val Val Gly Ala Met Gln Leu Tyr Ser Val Asp Arg Lys Val Ser Gln
            180                 185                 190

Pro Ile Glu Gly His Ala Ala Ser Phe Ala Gln Phe Lys Met Glu Gly
        195                 200                 205

Asn Ala Glu Glu Ser Thr Leu Phe Cys Phe Ala Val Arg Gly Gln Ala
    210                 215                 220

Gly Gly Lys Leu His Ile Ile Glu Val Gly Thr Pro Pro Thr Gly Asn
225                 230                 235                 240

Gln Pro Phe Pro Lys Lys Ala Val Asp Val Phe Phe Pro Pro Glu Ala
                245                 250                 255
Gln Asn Asp Phe Pro Val Ala Met Gln Ile Ser Glu Lys His Asp Val
            260                 265                 270
Val Phe Leu Ile Thr Lys Tyr Gly Tyr Ile His Leu Tyr Asp Leu Glu
        275                 280                 285
Thr Gly Thr Cys Ile Tyr Met Asn Arg Ile Ser Gly Glu Thr Ile Phe
    290                 295                 300
Val Thr Ala Pro His Glu Ala Thr Ala Gly Ile Ile Gly Val Asn Arg
305                 310                 315                 320
Lys Gly Gln Val Leu Ser Val Cys Val Glu Glu Glu Asn Ile Ile Pro
                325                 330                 335
Tyr Ile Thr Asn Val Leu Gln Asn Pro Asp Leu Ala Leu Arg Met Ala
            340                 345                 350
Val Arg Asn Asn Leu Ala Gly Ala Glu Glu Leu Phe Ala Arg Lys Phe
        355                 360                 365
Asn Ala Leu Phe Ala Gln Gly Asn Tyr Ser Glu Ala Ala Lys Val Ala
    370                 375                 380
Ala Asn Ala Pro Lys Gly Ile Leu Arg Thr Pro Asp Thr Ile Arg Arg
385                 390                 395                 400
Phe Gln Ser Val Pro Ala Gln Pro Gly Gln Thr Ser Pro Leu Leu Gln
                405                 410                 415

Tyr Phe Gly Ile Leu Leu Asp Gln Gly Gln Leu Asn Lys Tyr Glu Ser
            420                 425                 430

Leu Glu Leu Cys Arg Pro Val Leu Gln Gln Gly Arg Lys Gln Leu Leu
        435                 440                 445

Glu Lys Trp Leu Lys Glu Asp Lys Leu Glu Cys Ser Glu Glu Leu Gly
    450                 455                 460

Asp Leu Val Lys Ser Val Asp Pro Thr Leu Ala Leu Ser Val Tyr Leu
465                 470                 475                 480

Arg Ala Asn Val Pro Asn Lys Val Ile Gln Cys Phe Ala Glu Thr Gly
                485                 490                 495

Gln Val Gln Lys Ile Val Leu Tyr Ala Lys Lys Val Gly Tyr Thr Pro
            500                 505                 510

Asp Trp Ile Phe Leu Leu Arg Asn Val Met Arg Ile Ser Pro Asp Gln
        515                 520                 525

Gly Gln Gln Phe Ala Gln Met Leu Val Gln Asp Glu Glu Pro Leu Ala
    530                 535                 540

Asp Ile Thr Gln Ile Val Asp Val Phe Met Glu Tyr Asn Leu Ile Gln
545                 550                 555                 560

Gln Cys Thr Ala Phe Leu Leu Asp Ala Leu Lys Asn Asn Arg Pro Ser
                565                 570                 575

Glu Gly Pro Leu Gln Thr Arg Leu Leu Glu Met Asn Leu Met His Ala
            580                 585                 590

Pro Gln Val Ala Asp Ala Ile Leu Gly Asn Gln Met Phe Thr His Tyr
        595                 600                 605

Asp Arg Ala His Ile Ala Gln Leu Cys Glu Lys Ala Gly Leu Leu Gln
    610                 615                 620

Arg Ala Leu Glu His Phe Thr Asp Leu Tyr Asp Ile Lys Arg Ala Val
625                 630                 635                 640

Val His Thr His Leu Leu Asn Pro Glu Trp Leu Val Asn Tyr Phe Gly
                645                 650                 655

Ser Leu Ser Val Glu Asp Ser Leu Glu Cys Leu Arg Ala Met Leu Ser
            660                 665                 670

Ala Asn Ile Arg Gln Asn Leu Gln Ile Cys Val Gln Val Ala Ser Lys
        675                 680                 685

Tyr His Glu Gln Leu Ser Thr Gln Ser Leu Ile Glu Leu Phe Glu Ser
    690                 695                 700

Phe Lys Ser Phe Glu Gly Leu Phe Tyr Phe Leu Gly Ser Ile Val Asn
705                 710                 715                 720

Phe Ser Gln Asp Pro Asp Val His Phe Lys Tyr Ile Gln Ala Ala Cys
                725                 730                 735

Lys Thr Gly Gln Ile Lys Glu Val Glu Arg Ile Cys Arg Glu Ser Asn
            740                 745                 750

Cys Tyr Asp Pro Glu Arg Val Lys Asn Phe Leu Lys Glu Ala Lys Leu
        755                 760                 765

Thr Asp Gln Leu Pro Leu Ile Ile Val Cys Asp Arg Phe Asp Phe Val
    770                 775                 780

His Asp Leu Val Leu Tyr Leu Tyr Arg Asn Asn Leu Gln Lys Tyr Ile
785                 790                 795                 800

Glu Ile Tyr Val Gln Lys Val Asn Pro Ser Arg Leu Pro Val Val Ile
                805                 810                 815

Gly Gly Leu Leu Asp Val Asp Cys Ser Glu Asp Val Ile Lys Asn Leu
            820                 825                 830

Ile Leu Val Val Arg Gly Gln Phe Ser Thr Asp Glu Leu Val Ala Glu
        835                 840                 845

Val Glu Lys Arg Asn Arg Leu Lys Leu Leu Leu Pro Trp Leu Glu Ala
    850                 855                 860

Arg Ile His Glu Gly Cys Glu Glu Pro Ala Thr His Asn Ala Leu Ala
865                 870                 875                 880

Lys Ile Tyr Ile Asp Ser Asn Asn Asn Pro Glu Arg Phe Leu Arg Glu
                885                 890                 895

Asn Pro Tyr Tyr Asp Ser Arg Val Val Gly Lys Tyr Cys Glu Lys Arg
            900                 905                 910

Asp Pro His Leu Ala Cys Val Ala Tyr Glu Arg Gly Gln Cys Asp Leu
        915                 920                 925

Glu Leu Ile Asn Val Cys Asn Glu Asn Ser Leu Phe Lys Ser Leu Ser
    930                 935                 940

Arg Tyr Leu Val Arg Arg Lys Asp Pro Glu Leu Trp Gly Ser Val Leu
945                 950                 955                 960

Leu Glu Ser Asn Pro Tyr Arg Arg Pro Leu Ile Asp Gln Val Val Gln
                965                 970                 975

Thr Ala Leu Ser Glu Thr Gln Asp Pro Glu Glu Val Ser Val Thr Val
            980                 985                 990

Lys Ala Phe Met Thr Ala Asp Leu Pro Asn Glu Leu Ile Glu Leu Leu
        995                 1000                1005

Glu Lys Ile Val Leu Asp Asn Ser Val Phe Ser Glu His Arg Asn
    1010                1015                1020

Leu Gln Asn Leu Leu Ile Leu Thr Ala Ile Lys Ala Asp Arg Thr
    1025                1030                1035

Arg Val Met Glu Tyr Ile Asn Arg Leu Asp Asn Tyr Asp Ala Pro
    1040                1045                1050

Asp Ile Ala Asn Ile Ala Ile Ser Asn Glu Leu Phe Glu Glu Ala
    1055                1060                1065

Phe Ala Ile Phe Arg Lys Phe Asp Val Asn Thr Ser Ala Val Gln
    1070                1075                1080

Val Leu Ile Glu His Ile Gly Asn Leu Asp Arg Ala Tyr Glu Phe
    1085                1090                1095

Ala Glu Arg Cys Asn Glu Pro Ala Val Trp Ser Gln Leu Ala Lys
    1100                1105                1110

Ala Gln Leu Gln Lys Gly Met Val Lys Glu Ala Ile Asp Ser Tyr
    1115                1120                1125

Ile Lys Ala Asp Asp Pro Ser Ser Tyr Met Glu Val Val Gln Ala
    1130                1135                1140

Ala Asn Thr Ser Gly Asn Trp Glu Glu Leu Val Lys Tyr Leu Gln
    1145                1150                1155

Met Ala Arg Lys Lys Ala Arg Glu Ser Tyr Val Glu Thr Glu Leu
    1160                1165                1170

Ile Phe Ala Leu Ala Lys Thr Asn Arg Leu Ala Glu Leu Glu Glu
    1175                1180                1185

Phe Ile Asn Gly Pro Asn Asn Ala His Ile Gln Gln Val Gly Asp
    1190                1195                1200

Arg Cys Tyr Asp Glu Lys Met Tyr Asp Ala Ala Lys Leu Leu Tyr
    1205                1210                1215

Asn Asn Val Ser Asn Phe Gly Arg Leu Ala Ser Thr Leu Val His
    1220                1225                1230

Leu Gly Glu Tyr Gln Ala Ala Val Asp Gly Ala Arg Lys Ala Asn
    1235                1240                1245

Ser Thr Arg Thr Trp Lys Glu Val Cys Phe Ala Cys Val Asp Gly
    1250                1255                1260

Lys Glu Phe Arg Leu Ala Gln Met Cys Gly Leu His Ile Val Val
    1265                1270                1275

His Ala Asp Glu Leu Glu Glu Leu Ile Asn Tyr Tyr Gln Asp Arg
    1280                1285                1290

Gly Tyr Phe Glu Glu Leu Ile Thr Met Leu Glu Ala Ala Leu Gly
    1295                1300                1305

Leu Glu Arg Ala His Met Gly Met Phe Thr Glu Leu Ala Ile Leu
    1310                1315                1320

Tyr Ser Lys Phe Lys Pro Gln Lys Met Arg Glu His Leu Glu Leu
    1325                1330                1335

Phe Trp Ser Arg Val Asn Ile Pro Lys Val Leu Arg Ala Ala Glu
    1340                1345                1350

Gln Ala His Leu Trp Ala Glu Leu Val Phe Leu Tyr Asp Lys Tyr
    1355                1360                1365

Glu Glu Tyr Asp Asn Ala Ile Ile Thr Met Met Asn His Pro Thr
    1370                1375                1380

Asp Ala Trp Lys Glu Gly Gln Phe Lys Asp Ile Ile Thr Lys Val
    1385                1390                1395

Ala Asn Val Glu Leu Tyr Tyr Arg Ala Ile Gln Phe Tyr Leu Glu
    1400                1405                1410

Phe Lys Pro Leu Leu Leu Asn Asp Leu Leu Met Val Leu Ser Pro
    1415                1420                1425

Arg Leu Asp His Thr Arg Ala Val Asn Tyr Phe Ser Lys Val Lys
    1430                1435                1440

Gln Leu Pro Leu Val Lys Pro Tyr Leu Arg Ser Val Gln Asn His
    1445                1450                1455

Asn Asn Lys Ser Val Asn Glu Ser Leu Asn Asn Leu Phe Ile Thr
    1460                1465                1470

Glu Glu Asp Tyr Gln Ala Leu Arg Thr Ser Ile Asp Ala Tyr Asp
    1475                1480                1485

Asn Phe Asp Asn Ile Ser Leu Ala Gln Arg Leu Glu Lys His Glu
    1490                1495                1500

Leu Ile Glu Phe Arg Arg Ile Ala Ala Tyr Leu Phe Lys Gly Asn
    1505                1510                1515

Asn Arg Trp Lys Gln Ser Val Glu Leu Cys Lys Lys Asp Ser Leu
    1520                1525                1530

Tyr Lys Asp Ala Met Gln Tyr Ala Ser Glu Ser Lys Asp Thr Glu
    1535                1540                1545

Leu Ala Glu Glu Leu Leu Gln Trp Phe Leu Gln Glu Glu Lys Arg
    1550                1555                1560

Glu Cys Phe Gly Ala Cys Leu Phe Thr Cys Tyr Asp Leu Leu Arg
    1565                1570                1575

Pro Asp Val Val Leu Glu Thr Ala Trp Arg His Asn Ile Met Asp
    1580                1585                1590

Phe Ala Met Pro Tyr Phe Ile Gln Val Met Lys Glu Tyr Leu Thr
    1595                1600                1605

Lys Val Asp Lys Leu Asp Ala Ser Glu Ser Leu Arg Lys Glu Glu
    1610                1615                1620

Glu Gln Ala Thr Glu Thr Gln Pro Ile Val Tyr Gly Gln Pro Gln
    1625                1630                1635

Leu Met Leu Thr Ala Gly Pro Ser Val Ala Val Pro Pro Gln Ala
    1640                1645                1650

Pro Phe Gly Tyr Gly Tyr Thr Ala Pro Pro Tyr Gly Gln Pro Gln
    1655                1660                1665

Pro Gly Phe Gly Tyr Ser Met
    1670                1675

<210> 3
<211> 18
<212> PRT
<213> Homo sapiens

<400> 3

Leu His Ile Ile Glu Val Gly Thr Pro Pro Thr Gly Asn Gln Pro Phe
1               5                   10                  15

Pro Lys

<210> 4
<211> 12
<212> PRT
<213> Homo sapiens

<400> 4

Ile Val Leu Asp Asn Ser Val Phe Ser Glu His Arg
1               5                   10

<210> 5
<211> 9
<212> PRT
<213> Homo sapiens

<400> 5

Val Ala Asn Val Glu Leu Tyr Tyr Arg
1               5

<210> 6
<211> 10
<212> PRT

<213>  Homo sapiens

<400>  6

Gln Leu Pro Leu Val Lys Pro Tyr Leu Arg
1               5                   10


<210>  7
<211>  11
<212>  PRT
<213>  Homo sapiens

<400>  7

Val Asp Lys Leu Asp Ala Ser Glu Ser Leu Arg
1               5                   10


<210>  8
<211>  9
<212>  PRT
<213>  artificial sequence

<220>
<223>  flag

<400>  8

Gly Asp Tyr Lys Asp Asp Asp Asp Lys
1               5


<210>  9
<211>  1182
<212>  DNA
<213>  Homo sapiens

<400>  9
atggaggagc cgcagtcaga tcctagcgtc gagccccctc tgagtcagga aacattttca      60

gacctatgga aactacttcc tgaaaacaac gttctgtccc ccttgccgtc ccaagcaatg     120

gatgatttga tgctgtcccc ggacgatatt gaacaatggt tcactgaaga cccaggtcca     180

gatgaagctc ccagaatgcc agaggctgct ccccgcgtgg cccctgcacc agcagctcct     240

acaccggcgg cccctgcacc agccccctcc tggcccctgt catcttctgt cccttcccag 300

aaaacctacc agggcagcta cggtttccgt ctgggcttct tgcattctgg gacagccaag 360

tctgtgactt gcacgtactc ccctgccctc aacaagatgt tttgccaact ggccaagacc 420

tgccctgtgc agctgtgggt tgattccaca cccccgcccg gcacccgcgt ccgcgccatg 480

gccatctaca agcagtcaca gcacatgacg gaggttgtga ggcgctgccc ccaccatgag 540

cgctgctcag atagcgatgg tctggcccct cctcagcatc ttatccgagt ggaaggaaat 600

ttgcgtgtgg agtatttgga tgacagaaac acttttcgac atagtgtggt ggtgccctat 660

gagccgcctg aggttggctc tgactgtacc accatccact acaactacat gtgtaacagt 720

tcctgcatgg gcggcatgaa ccggaggccc atcctcacca tcatcacact ggaagactcc 780

agtggtaatc tactgggacg gaacagcttt gaggtgcatg tttgtgcctg tcctgggaga 840

gaccggcgca cagaggaaga gaatctccgc aagaaagggg agcctcacca cgagctgccc 900

ccagggagca ctaagcgagc actgcccaac aacaccagct cctctcccca gccaaagaag 960

aaaccactgg atggagaata tttcaccctt cagatccgtg ggcgtgagcg cttcgagatg 1020

ttccgagagc tgaatgaggc cttggaactc aaggatgccc aggctgggaa ggagccaggg 1080

gggagcaggg ctcactccag ccacctgaag tccaaaaagg gtcagtctac ctcccgccat 1140

aaaaaactca tgttcaagac agaagggcct gactcagact ga 1182

<210> 10
<211> 465
<212> DNA
<213> Homo sapiens

<400> 10
ccatttcctc agagaacacg gcccccttgg gccaaaagga catgaagaag ctcttgctaa 60

tgccagcctg gctctcctca tcccgccccc tgcacccctg cccttctggc tgccctccct 120

tctcctagct ctgtcccctc tcacttcagg agtctcaagt ccttcagact actccaaagt 180

cgggggatct ctggatgggt aggaggtgat ctcaccgcct cctctcttgc ccgggcttgt 240

cgagatgaac ttcctgatgc tggcggcgct gaagctgaca ctagcggggg cacctccctg 300

acatgaacgc ccctcgagac tgggccagtg ctcctgatgc ctgggcacct gcggaaaggc 360

acccagcgtg gccgccgtgg catgccttga gtgtgtgggt ggggactgtt gcaaactgac 420

attccagctg tcccagtcca ttttgtgctg ctctcacaca acccc 465

<210> 11
<211> 5028
<212> DNA
<213> Homo sapiens

<400> 11
atggcccaga ttctgccaat tcgttttcag gagcatctcc agctccagaa cctgggtatc 60

aacccagcaa acattggctt cagtaccctg actatggagt ctgacaaatt catctgcatt 120

agagaaaaag taggagagca ggcccaggtg gtaatcattg atatgaatga cccaagtaat 180

ccaattcgaa gaccaatttc agcagacagc gccatcatga atccagctag caaagtaatt 240

gcactgaaag ctgggaaaac tcttcagatt tttaacattg aaatgaaaag taaaatgaag 300

gctcatacca tgactgatga tgtcaccttt tggaaatgga tctctttgaa tacggttgct 360

cttgttacgg ataatgcagt ttatcactgg agtatggaag gagagtctca gccagtgaaa 420

atgtttgatc gccattctag ccttgcaggg tgccagatta tcaattaccg tacagatgca 480

aaacaaaagt ggttacttct gactggtata tctgcacagc aaaatcgtgt ggtgggagct 540

atgcagctat attctgtaga taggaaagtg tctcagccca ttgaaggaca tgcagctagc 600

tttgcacagt ttaagatgga aggaaatgca gaagaatcaa cgttattttg ttttgcagtt 660

cggggccaag ctggagggaa gttacatatt attgaagttg gcacaccacc tacagggaac 720

cagccctttc caaagaaggc agtggatgtc ttctttcctc cagaagcaca aaatgatttt 780

cctgttgcaa tgcagatcag tgaaaagcat gatgtggtgt tcttgataac caagtatggt 840

tatatccacc tctatgatct tgagactggt acctgcatct acatgaatag aatcagtgga 900

gaaacaattt ttgttactgc acctcatgaa gccacagctg gaataattgg agtaaacaga 960

aagggacaag ttctgtcagt gtgtgtggaa gaagaaaaca taattcctta catcaccaat 1020

gttctacaaa atcctgattt ggctctgaga atggctgtac gtaataactt agccggtgct 1080

gaagaactct ttgcccggaa atttaatgct ctttttgccc agggaaatta ctcggaggca 1140

gcaaaggtgg ctgctaatgc accaaaggga attcttcgta ctccagacac tatccgtcgg 1200

ttccagagtg tcccagccca gccaggtcaa acttctcctc tacttcagta ctttggtatc 1260

cttttggacc agggacagct caacaaatac gaatccttag agctttgtag gcctgtactt 1320

cagcaagggc gaaaacagct tttggagaaa tggttaaaag aagataagct ggaatgttct 1380

gaagaactgg gtgatcttgt gaaatctgtg gaccctacat tggcacttag tgtgtaccta 1440

agggctaacg tcccaaataa agtcattcag tgctttgcag aaacaggtca agtccaaaag 1500

attgttttat atgctaaaaa agttggatac actccagatt ggatatttct gctgagaaat 1560

gtaatgcgaa tcagtccaga tcagggacag cagtttgccc aaatgttagt tcaagatgaa 1620

gagcctcttg ctgacatcac acagattgta gatgtcttta tggaatacaa tctaattcag 1680

cagtgtactg cattcttgct tgatgctctg aagaataatc gcccatctga aggtccttta 1740

cagacgcggt tacttgagat gaaccttatg catgcgcctc aagttgcaga tgctattcta 1800

ggcaatcaga tgttcacaca ttatgaccgg gctcatattg ctcaactgtg tgaaaaggct 1860

ggcctactgc agcgtgcatt agaacatttc actgatttat atgatataaa acgtgcagtg 1920

gttcacaccc atcttcttaa ccctgagtgg ttagtcaact actttggttc cttatcagta 1980

gaagactccc tagaatgtct cagagccatg ctgtctgcca acatccgtca gaatctgcag 2040

atttgtgttc aggtggcttc taaatatcat gaacaactgt caactcagtc tctgattgaa 2100

ctttttgaat ctttcaagag ttttgaaggt ctcttttatt ttctgggatc cattgttaac 2160

tttagccagg acccagatgt gcactttaaa tatattcagg cagcttgcaa gactgggcaa 2220

atcaaagaag tagaaagaat ctgtagagaa agcaactgct acgatcctga gcgagtcaag 2280

aattttctta aggaagcaaa actaacagat cagctaccac ttatcattgt gtgtgatcga 2340

tttgactttg tccatgattt ggtgctctat ttatatagaa ataatcttca aaagtatata 2400

gagatatatg tacagaaggt gaatccaagt cgacttcctg tagttattgg aggattactt 2460

gatgttgact gttctgaaga tgtcataaaa aacttgattc ttgttgtaag aggtcaattc 2520

tctactgatg agcttgttgc tgaggttgaa aaaagaaaca gattgaaact gcttctgcct 2580

tggctagagg ccagaattca tgagggctgt gaggagcctg ctactcacaa tgccttagcc 2640

aaaatctaca tagacagtaa taacaacccg gagagatttc ttcgtgaaaa tccctactat 2700

gacagtcgcg ttgttggaaa gtattgtgag aagagagatc cacatctggc ctgtgttgct 2760

tatgaacgtg gccaatgtga tctggaactt attaatgttt gcaatgagaa ttccctcttc 2820

aaaagtcttt ctcgctacct ggtacgtcga aaggatccag aattgtgggg cagcgtgctg 2880

ctggaaagca atccttacag gagaccccta attgaccagg ttgtacaaac agctttgtct 2940

gagactcagg accctgaaga agtgtcagta actgtaaagg ctttcatgac tgcagacctt 3000

cctaatgaac tcattgaact gctggagaaa attgtccttg ataactctgt attcagtgaa 3060

cacaggaatc tgcaaaacct ccttatcctc actgcaatta aggctgaccg tacacgtgtt 3120

atggagtata ttaaccgcct ggataattat gatgccccag atattgccaa tatcgccatc 3180

agcaatgagc tgtttgaaga agcatttgcc attttccgga aatttgatgt caatacttca 3240

gcagttcagg tcttaattga gcatattgga aacttggatc gggcatatga gtttgctgaa 3300

cgttgcaatg aacctgcggt ctggagtcaa cttgcaaaag cccagttgca gaaaggaatg 3360

gtgaaagaag ccattgattc ttatatcaaa gcagatgatc cttcctccta catggaagtt 3420

gttcaggctg ccaatactag tggaaactgg gaagaactgg tgaagtactt gcagatggcc 3480

cgtaagaagg ctcgagagtc ctatgtggag acagaactga tattcgcact ggctaaaaca 3540

aaccgccttg cagagttaga agaatttatc aatggaccaa ataatgctca tatccaacaa 3600

gttggtgacc gttgttatga tgaaaaaatg tatgatgctg ctaagttgtt gtacaataat 3660

gtttccaatt ttggacgttt ggcatctacc ctggttcacc tgggtgaata tcaggcagct 3720

gttgatgggg ctaggaaagc taacagtact cgaacatgga aagaggtctg cttcgcctgt 3780

gtagatggga aagaattccg tcttgctcag atgtgtggac ttcatattgt tgtacatgca 3840

gatgaattag aagaacttat caactactat caggatcgtg gctattttga agagctgatc 3900

accatgttgg aagcagcact gggacttgag cgagctcaca tgggaatgtt tactgaatta 3960

gctattctat actctaaatt taagcctcag aaaatgaggg agcacctgga gctgttctgg 4020

tctagagtga atattcccaa ggtgctaaga gctgcagaac aagctcatct ttgggcagaa 4080

ctggtgtttt tgtatgacaa gtatgaagaa tatgataatg ccataattac catgatgaat 4140

catccaactg atgcctggaa agaagggcaa ttcaaagata tcattaccaa ggttgccaat 4200

gtggaactat actacagagc aatacagttc tacttagaat tcaagcctct gttgttaaat 4260

gatttgctga tggtgctgtc tccacggttg gatcacactc gtgcagtcaa ttatttcagc 4320

aaggttaaac agctaccact ggtgaaaccg tatttgcgtt cagttcagaa ccataacaac 4380

aaatctgtga atgaatcatt gaacaatctt tttattacag aagaagatta tcaggctctg 4440

cgaacatcaa tagatgctta tgacaacttt gacaatatct cgcttgctca gcgtttggaa 4500

aaacatgaac tcattgagtt caggagaatt gctgcttatc tcttcaaagg caacaatcgc 4560

tggaaacaga gtgtagagct gtgcaagaaa gacagccttt acaaggatgc aatgcagtat 4620

gcttctgaat ctaaagatac tgaattggct gaagaactcc tgcagtggtt tttgcaggaa 4680

gaaaaaagag agtgctttgg agcttgtctg tttacctgtt acgatctttt aaggccagat 4740

gtcgtcctag aaactgcatg gaggcacaat atcatggatt ttgccatgcc ctatttcatc 4800

caggtcatga aggagtactt gacaaaggtg gataaattag atgcttcaga atcactgaga 4860

aaagaagaag aacaagctac agagacacaa cccattgttt atggtcagcc ccagttgatg 4920

ctgacagcag gacccagtgt tgccgtccct ccccaggcac cttttggtta tggttatacc 4980

gcaccaccgt atggacagcc acagcctggc tttgggtaca gcatgtga 5028

## Description

### Technical field of the invention:

The present invention relates to a transcriptional factor inducing apoptosis of cancer cells.

### Prior Art:

A tumor suppressor gene p53 is a transcription factor which specifically binds to DNA sequences of target genes such as p21, p53R2 or p53AIP1 and controls transcriptional activity of these genes. Therefore the mechanism of cellular tumorigenesis involving p53 has been extensively studied (Cell Technology vol. 22, No.1, 23-28 (2003)). Among various target genes controlling p53, p53AIP1 is a protein localized to mitochodria, which controls mitochondrial membrane potential and releases cytochrome c, and, therefore, has a function to induce positive apoptotic effect (Oda K et al., Cell, vol. 102, 849-862, 2000; Matsuda K et al., Cancer Res., vol. 62, 2883-2889, 2002). The p53AIP1 has a strong correlation to phosphorylation of Ser46, which has an important role in p53-dependent induction of apoptosis (Japanese Patent Application No. 2001-292953), and the expression of p53AIP1 gene induces apoptosis of cancer cells. For example, when cells are exposed to a strong stress such as generation of DNA damage, it is believed that Ser46 of transcriptional factor p53 is phosphorylated by the function of p53DINP1 and so forth, the expression of p53AIP1 is enhanced and apoptosis of cancer cells is induced (Cell Technology vol. 22, No.1, 23-28 (2003)).

On the other hand, to induce apoptosis of cancer cells, various anticancer drugs such as cisplatin have been used and most of them induce apoptosis by generating DNA damage in cancer cells and in normal cells, which sometimes leads to side effects. Radiation therapy of cancer also induces side effects by generating DNA damage in normal cells.
Furthermore, attempts to induce apoptosis and to kill cancer cells by transfecting cancer cells with adenovirus vectors containing p53 gene, or Bax or p53AIP1, inducible by p53, have been tried but not been successful.

### Problems to be solved by the Invention:

The purpose of the present invention is to provide a new method for cancer treatment by inducing apoptosis exclusively in cancer cells and by killing them.

### Means to solve the Problems:

The inventors have been studied in solving above-described purpose and found that an about 170 kDa protein, probably attributable to clathrin heavy chains, was co-precipitated with p53 as p53 was expressed in cancer cells and then that the transcriptional activity of p53AIP1 promoter was enhanced when an expression vector carrying cDNA of clathrin heavy chain were transfected into nuclei of cancer cells. Based on these findings, the inventors concluded that the clathrin heavy chains and p53 are associated to compose a transcriptional factor and the transcriptional factor enhances the transcriptional activity of p53AIP1 promoter and induces apoptosis of cancer cells.

That is, the present invention is a transcriptional factor, comprising p53 (SEQ ID NO:1) or a mutated type p53, wherein one or more amino acids are deleted, substituted or added with respect to the amino sequence of p53, and clathrin heavy chains (SEQ ID NO:2) and having an activity to induce apoptosis of cancer cells. These proteins could be extracted and purified from tissues or cells of human and others, or could be obtained from transformants having the DNA coding these proteins.
Moreover, said mutated p53 could be p53, wherein at least the serine residue at amino acid position 46 (hereinafter abbr. as Ser46) is substituted, preferably Ser46 is substituted to Phe.
Furthermore, the present invention is a cancer treatment drug, comprising the transcriptional factor as an effective component.

### Brief Explanation of the Drawings:

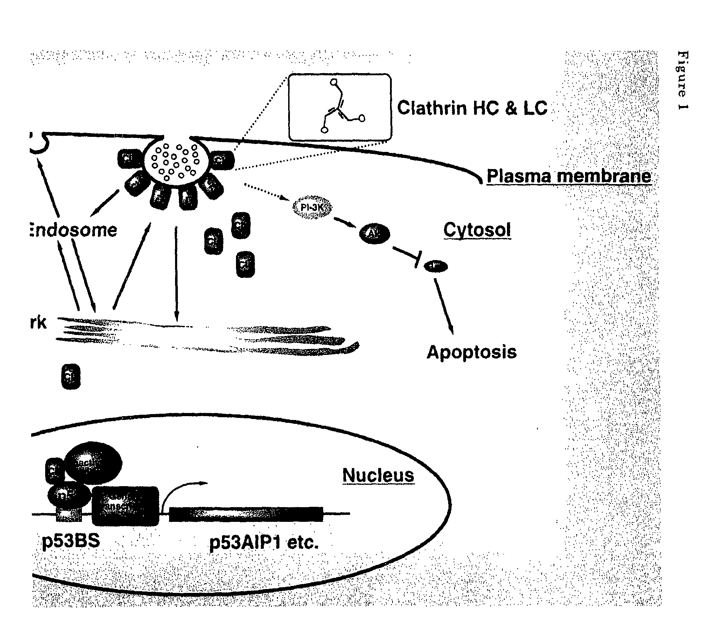

Figure 1 shows a conceptual diagram showing the transcriptional control of p53AIP1 by clathrin heavy chains.
Figure 2 shows the structure of pc-p53-f vector.
Figure 3 shows the structure of p53AIP1.pro.reporter vector.
Figure 4 shows the structure of pc-CHC vector.
Figure 5 shows the transcriptional activity of p53AIP1 in human osteosarcoma-derived cells, transfected with the expression vector (pcDNA-p53-f).
Figure 6 shows the SDS-PAGE (silver stained) of the cell lysates from human non-small cell lung cancer-derived cells transfected with the expression vector (pcDNA3.1 or pcDNA-p53-f).
Figure 7 shows the Western blotting, detected by anti-clathrin heavy chain antibody and anti-p53 antibody, of the cell lysates from human non-small cell lung cancer-derived cells, transfected with the expression vector (pcDNA-p53-f). In the figure, WT, 46A, 46D, 46F and Δ44-63 indicate WT-p53, S46A-p53, S46D-p53, S46F-p53 and 44-63-p53, respectively.
Figure 8 shows the Western blotting, detected by anti-clathrin heavy chain antibody, of the cytoplasmic and nuclear extracts from human non-small cell lung cancer-derived cells, transfected with the vector (pcDNA3.1, pcDNA-p53-f). In the figure, pcDNA, WT, 46F and Δ44-63 indicate pcDNA3.1, WT-p53, S46F-p53, 44-63-p53, respectively.
Figure 9 shows the relative transcriptional activation in the cell lysates from human osteosarcoma-derived cells transfected with both the expression vector (pc-CHC) containing cDNA of clathrin heavy chain and the p53 expression vector (pcDNA-p53-f). In the figure, clathrin HC, WT, S46F and Δ44-63 indicate pc-CHC, WT-p53, S46F-p53 and 44-63-p53, respectively.

### Detailed Description of the Invention

Clathrin has heavy and light chains and, generally, functions for endocytosis, i.e. import of materials from cell surface into cytoplasm. However, the present inventors found that the clathrin heavy chains do not associate with light chains in cancer cells but bind to p53, enhance the transcriptional activity of p53 and induce apoptosis. Clathrin was found to bind strongly to the p53, whose S46 is substituted to phenylalanine, and to induce strong apoptosis.
By injecting the transcriptional factor of the present invention or injecting only clathrin heavy chains into cancer cells by some kind of methods, these bind to p53 inside cancer cells, which induce apoptosis of cancer cells, then kill them.
Using such transcriptional factor would enable specific induction of apoptosis of cancer cells and their death without damaging normal cells more effectively than previous methods. This method, therefore, provides more effective methods for cancer treatment than previous ones.

The following Examples illustrate the invention, however, these are not constructed to limit the scope of the invention.

### Test Example 1

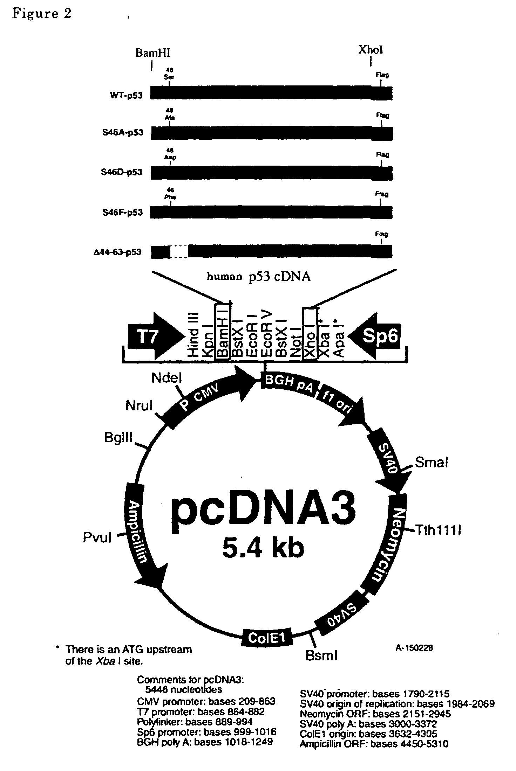

In this Test Example, the inventors prepared the following five kind of vectors (named generically as (pcDNA-p53-f]) used in the subsequent Examples.
(i) A wild type human p53 expression vector (hereinafter referred to as [WT-p53]) comprising wild type human p53 gene inserted in pcDNA3 plasmid (Invitrogen, Ref. Figure 2).
(ii) A mutated type human p53 expression vector (hereinafter referred to as [S46A-p53]) comprising the mutated p53, prepared by the substitution of Ser46 to Ala and inserted to pcDNA3 plasmid.
(iii) A mutated type human p53 expression vector (hereinafter referred to as [S46D-p53]) comprising the mutated p53, prepared by the substitution of Ser46 to Asp and inserted to pcDNA3 plasmid.
(iv) A mutated type human p53 expression vector (hereinafter referred to as [S46F-p53]) comprising the mutated p53, prepared by the substitution of Ser46 to Phe and inserted to pcDNA3 plasmid.
(v) A mutated human type p53 expression vector (hereinafter referred to as [44-63-p53]) comprising the mutated p53, prepared by deletion of amino acid position 44 to 63 and inserted to pcDNA3 plasmid.

### The above vectors were prepared according to the following procedures:

For preparation various pcDNA-p53-f, pcDNA3 plasmid (Invitrogen) was cleaved at the restriction sites XhoI and XbaI and inserted with the double-strand DNA (SEQ ID NO: 8) coding a Flag sequence (GDYKDDDDK) comprising of 9 amino acid residues (pcDNA-f). The prepared pcDNA3-f plasmid was cleaved at the restriction sites BamHI and XhoI and was inserted with wild or mutated type human p53 genes (SEQ ID NO:9) with BamHI and XhoI ends (pcDNA-p53-f). The maps of these vectors are shown in Figure 2.

### Test Example 2.

In this Test Example, the inventors prepared a reporter plasmid, wherein about 500 bp DNA containing p53 binding sequence in the intron 1 of p53AIP1 gene was inserted to the upstream of luciferase gene in pGL3-promoter vector (Promega).

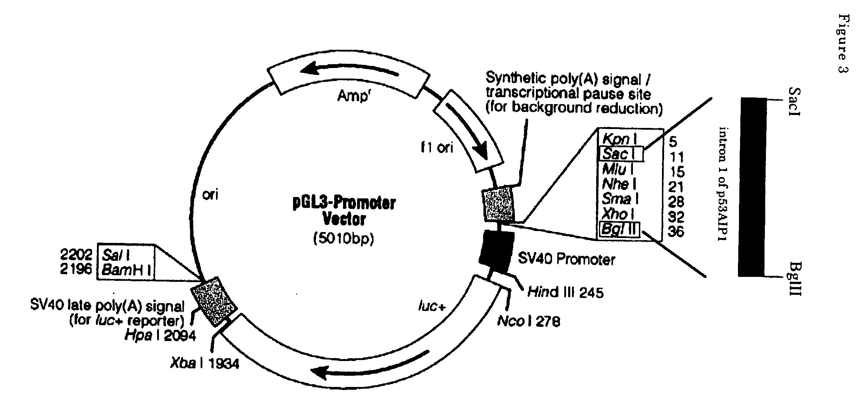

pGL3-promoter vector (Promega) was cleaved at the restriction sites SacI and BgIII and was inserted with p53AIP1 intron 1 (about 500 bp, SEQ ID NO: 10) containing a p53 binding sequence with SacI and BglII ends. The map of the vector is shown in Figure 3. The reporter is hereinafter referred to as [p53AIP1pro. reporter].

### Test Example 3

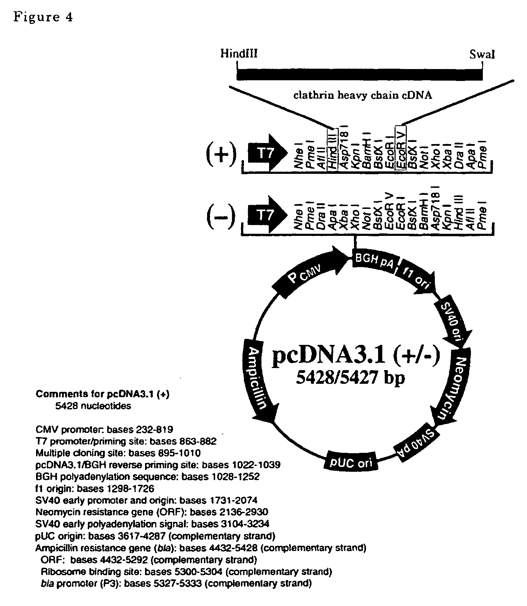

With the similar procedures as Test Example 1, clathrin heavy chain expression vector (hereinafter, referred to as [pc-CHC]) was prepared by insertion of clathrin heavy chain gene (gifted from Kazusa DNA Research Institute, SEQ ID NO: 11) to pcDNA3 plasmid (Invitrogen, Ref. Figure 2). The map of the vector is shown in Figure 4.

### Test Example 4.

In this Test Example, five kinds of expression vectors prepared in the Test Example 1 were transfected to human osteosarcoma-derived cells and the cell lysate was examined.

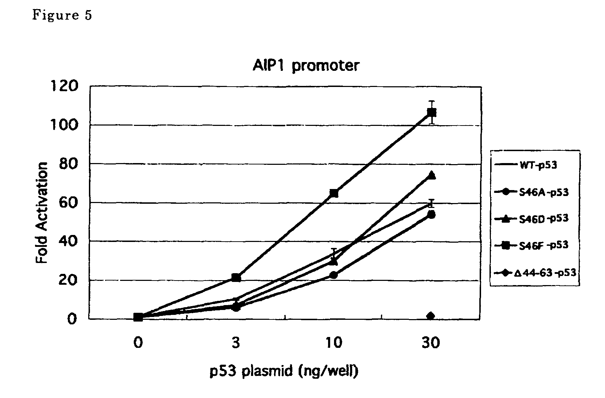

The examination was performed by the following procedures.
1) Saos-2 cells (osteosarcoma-derived) were seeded to 24 well tissue culture plate at 7 x 10⁴ cells/ well and cultured for over night.
2) The DNA to be transfected was prepared as shown in Table 1, by the use of pcDNA-p53-f and p53AIP1 pro.reporter prepared in Test Example 1 and 2. Total amount of DNA added was 30 ng with the addition of pcDNA3.1 to 0 ~30 ng of each pcDNA-p53-f. In Table 1, phRG-TK shows a plasmid (Promega, Inner control) expressing Renilla reniformis Luciferase. The method for transfection is according to the protocol attached to the kit of Invitrogen. 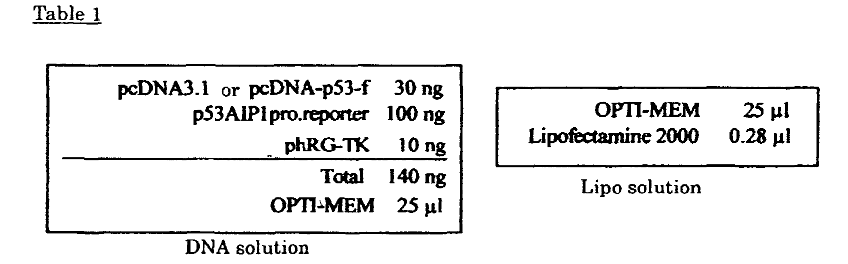
3) DNA solution and Lipo solution were mixed and incubated for 30 min at room temperature.
4) The DNA-liposome complex was dropped to cultures prepared in 1).
5) After 4 hours, DNA-liposome complex was removed and the culture was washed by 1 x PBS-.
6) At 24 hours after transfection, the culture was washed by 1 x PBS-, and Firefly and Renilla reniformis (the inner control) Luciferase activity was measured by luminometer, respectively. The method for the measurement was according to the protocol of Promega attached to the kit.
7) Relative fold activation was calculated by dividing the activity of Firefly Luciferase by that of Renilla reniformis Luciferase in the lysates from cells transfected with pcDNA 3.1. The results are shown in Figure 5. Ser46Phe substituent of p53 enhanced the transcription from p53AIP1 promoter much stronger than wild type p53.

### Example 1

In this Example, five kinds of expression vectors prepared in Test Examplel was transfected to human non-small cell lung cancer-derived cells and the cell lysate was examined.

The examination was according to the following procedures.
1) H1299 cells (non-small cell lung cancer-derived) were seeded to 15 cm culture dishes at 9 x 10⁶/ plate (10 plates) and cultured for over night.
2) The DNA to be transfected was prepared according to Table 2. The method of transfection was according to the protocol attached to the kit of Roche Applied Science. 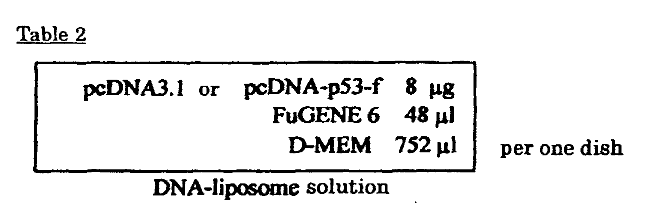
3) The DNA -liposome was incubated for 30 min at room temperature and dropped to the cultures prepared in 1).
4) At 21 hours after transfection, cells were split by a scraper and recovered by low speed centrifugation at 600 x g for 5 min.
5) The cells were washed once by 1 x PBS- and lysed by 10 ml of the cell lysis buffer shown in Table 3.

**Table 3**

| Cell lysis buffer | |
|---|---|
| 50 mM Tris-HCI (pH7.2) | 10 µg/ml antipain |
| 150 mM NaCl | 10 µg/ml pepstatin |
| 2 mM MgCl₂ | 10 µg/ml chymostatin |
| 0.1 mM EDTA | 10 µg/ml leupeptin |
| 0.1 mM EGTA | 10 µg/ml E64 |
| 0.5 mM DTT | 10 µg/ml PMSF |
| 1 mM Na₃V0₄ | 0.1 % NP-40 |
| 10 mM NaF | |

6) After the cells were sonicated, the insoluble fraction was removed by the
1) Cells in a 15 cm culture dish was transfected according to the procedure in Test Example 5.
2) After cell harvest, cells were suspended in 1 ml of the hypotonic buffer*1 shown in Table 4 and the plasma membrane was disrupted by a homogenizer.

**Table 4**

| Hypotonic buffer *1 | |
|---|---|
| 10 mM HEPES-KOH (pH7. 9) | 10 µg/ml antipain |
| 10 mM KCl | 10 µg/ml pepstatin |
| 1.5 mM MgCl₂ | 10 µg/ml chymostatin |
| 0.5 mM DTT | 10 µg/ml leupeptin |
| 1 mM Na ₃V0₄ | 10 µg/ml E64 |
| 50 mM NaF | 10 µg/ml PMSF |

| Hypertonic buffer*2 | |
|---|---|
| 25 mM HEPES-KOH (pH7.9) | 10 µg/ml antipain |
| 420 mM KCl | 10 µg/ml pepstatin |
| 10 % glycerol | 10 µg/ml chymostatin |
| 0.1 mM DTT | 10 µg/ml leupeptin |
| 1 mM Na₃V0₄ | 10 µg/ml E64 |
| 50 mM NaF | 10 µg/ml PMSF |

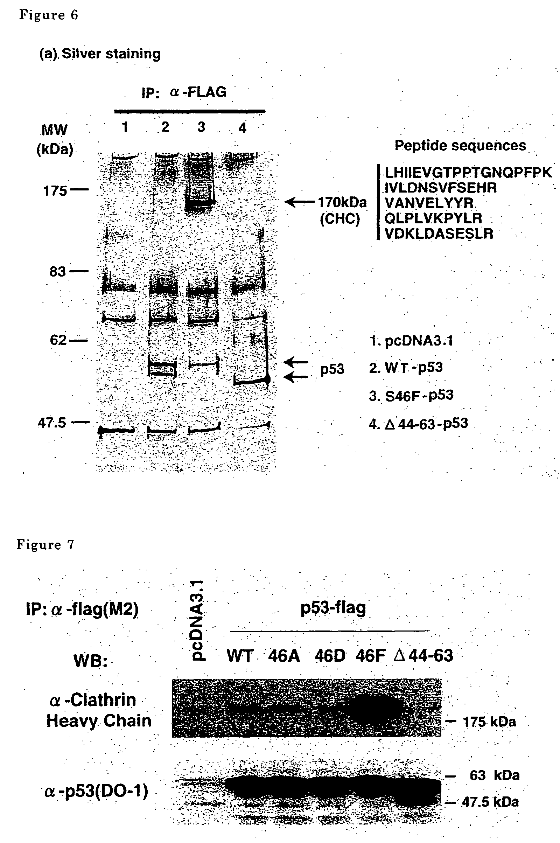

3) The cell debris was centrifuged at 600 x g, for 5 min at 4°C and nuclear fraction was separated from cytoplasmic fraction.
4) Nuclear fraction was sufficiently separated from cytoplasmic proteins by repeating the centrifugation in 2) and 3) for two times.
5) The nuclear fraction was suspended in 0.2 ml of the hypertonic buffer*2 in Table 4, kept on ice for 30 min and proteins were eluted from nuclei.
6) Insoluble fraction was removed from nuclear fraction by centrifugating at 10 k x g for 5 min at 4°C.
7) Proteins from cytoplasmic and nuclear fractions were Western blotted, i.e the proteins were loaded on SDS-PAGE, transferred to PVDF membrane and stained with anti-clathrin heavy chain antibody as described in Test Example 5. The
7) centrifugation at 15 krpm for 15 min at 4°C.
8) The supernatant was incubated with 0.2 ml of anti-FLAG-anti-agarose beads (Sigma) for over night.
9) After the incubation, beads were washed with the above cell lysis buffer for 4 times.
10) Then, p53 protein was eluted from the beads with the above cell lysis buffer containing 1.5 mg/ml FLAG peptide, competitively.
11) The eluted fraction was loaded on SDS-PAGE and stained with silver. The results were shown in Figure 6. AS the results, a 170 kDa protein was strongly co-precipitated with p53 in S46F substituent. Analysis by mass spectroscopy of the protein revealed the amino acid sequence of LHIIEVGTPPTGNQPFPK,
   IVLDNSVFSEHR, VANVELYYR, QLPLVKPYLR and VDKLDASESLR (SEQ ID NO: 3~7). These amino acid sequences correspond to amino acid position 228-245, 1011-1022, 1398-1406, 1444-1453 and 1610-1620 of clathrin heavy chain (SEQ ID NO:2). The results show that the 170 kDa protein is identical to clathrin heavy chain.
12) The identification of the 170 kD protein was performed after the protein was concentrated to 20 times more than the amount used for silver staining, loaded on SDS-PAGE, stained with CBB, cut out the target band, digested by trypsin and analyzed by use of mass spectroscopy.
13) Hundredth part of the eluate obtained in the procedure 10) was performed Western blotting, i.e. the eluate was loaded on SDS-PAGE, transferred to PVDF membrane and stained with anti-clathrin heavy chain antibody (Transduction Laboratories) or with anti-p53 antibody (Santa Cruz). In this Example, the bands were visualized by horseradish peroxidase-conjugated anti-mouse IgG secondary antibody (Amersham) and ECL kit. The results are shown in Figure 7. Clathrin heavy chains were found to bind strongly to S46Fsubstituent.

### Test Example 5

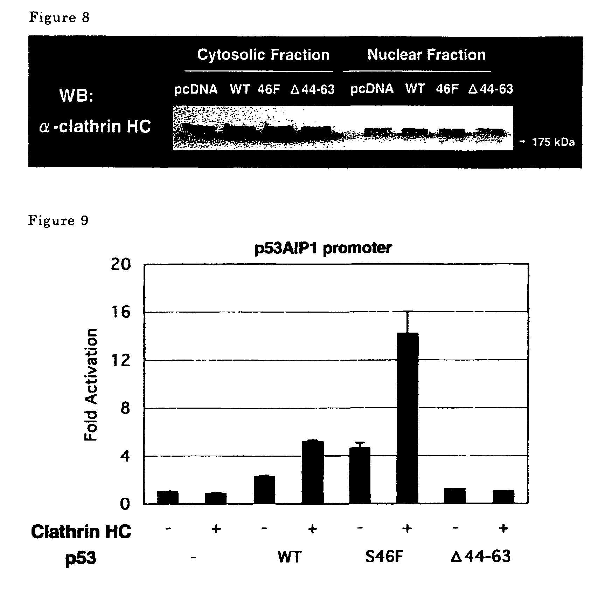

In this Test Example, nuclear extract from human non-small cell lung cancer-derived cells used in Test Example 5 was examined. The test was according to the following procedures. amount of proteins from nuclear fraction was five times more than that of cytoplasmic fraction. The results are shown in Figure 8. Previously, clathrin was considered to be localized only in cytoplasm, however, a part of clathrin was confirmed to be localized in nuclei.

### Example 2

In this Example, experiments were performed according to the procedure in test Example 4 except that the DNA in the DNA solution of 2) was prepared according to Table 5 and used pc-CHC prepared in Test Example 3 as the DNA transfected.

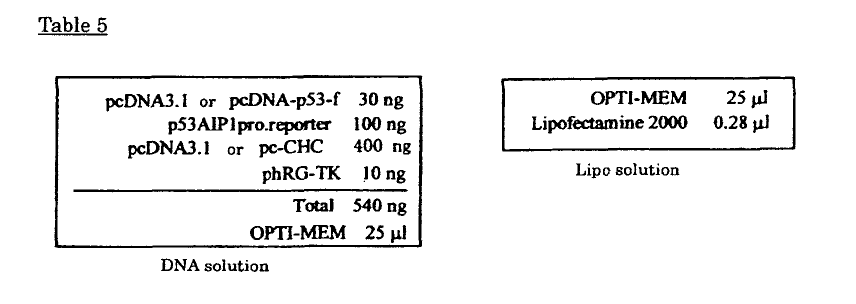

The results are shown in Figure 9. For the DNA solution, pc-CHC (400 ng) and pcDNA 3.1 (400 ng) solutions are referred to as clathrin + and clathrin -, respectively. Clathrin heavy chain cDNA, inserted to an expression vector and transfected together with p53, was confirmed to enhance the transcriptional activity of p53AIP1 promoter. Especially, the enhancing effect of S46F substituent was predominant.

## Claims

1. A transcriptional factor, comprising p53 or a mutated type p53, wherein one or more amino acids are deleted, substituted or added with respect to the amino sequence of p53, and clathrin heavy chains and having an activity to induce apoptosis of cancer cells.

2. The transcriptional factor of claim 1, wherein at least the serine residue at amino acid position 46 is substituted in said mutated type p53.

3. The transcriptional factor of claim 2, wherein said serine residue at amino acid position 46 is substituted to phenylalanine.

4. A cancer treatment drug comprising the transcriptional factor of any one of claims 1 to 3 as an effective component.

5. A method for treating cancer comprising injecting the transcriptional factor of any one of claims 1 to 3, or clathrin heavy chains to a cancer cell.
